# EUROPEAN PATENT SPECIFICATION

(11) **EP 1 973 490 B1**
(45) Date of publication and mention of the grant of the patent: **27.02.2019**
(21) Application number: 07718202.0
(22) Date of filing: 17.01.2007
(51) Int. Cl.: A61C 1/00, A61C 17/20

(54) **WIRELESS SYSTEM WITH FOOTSWITCH FOR OPERATING A DENTAL OR MEDICAL TREATMENT APPARATUS**
DRAHTLOSES SYSTEM MIT FUSSSCHALTER ZUR BEDIENUNG EINES DENTALEN ODER MEDIZINISCHEN BEHANDLUNGSGERÄTS
SYSTEME SANS FIL A INTERRUPTEUR AU PIED POUR ACTIONNER UN APPAREIL DE TRAITEMENT DENTAIRE OU MEDICAL

(30) Priority: 17.01.2006 US 333970
(43) Date of publication of application: 01.10.2008
(73) Proprietor: DENTSPLY SIRONA Inc., York, PA 17401-2991 (US)
(72) Inventor: LINT, Kevin, Seven Valleys, PA 17360 (US); KILE, Jeremy, Wrightsville, PA 17368 (US); GUARAGNO, Kenneth, R., Spring Grove, PA 17362 (US); REAGAN, Joseph, R., Steelton, PA 17113 (US)
(74) Representative: Hartz, Nikolai
(86) International application number: PCT/US2007/001443
(87) International publication number: WO 2007/084668

(56) References cited:
- WO-A-2005/053561
- US-A- 5 125 837
- US-A- 5 754 016
- US-A1- 2005 251 228

## Description

### BACKGROUND OF THE INVENTION

### Field of the Invention

The present invention relates generally to a footswitch system for operating a dental or medical treatment apparatus. The system includes a footswitch device and a dental/medical base unit, each having a communication element for transmitting and receiving signals. The footswitch can be tethered to the base unit by a connector cable in a hard-wired system. Alternatively, the footswitch can be used to remotely control the base unit in a wireless system. The footswitch device is particularly suitable for operating an ultrasonic dental scaler unit.

### Brief Description of the Related Art

Today, dental and medical professionals routinely use instruments that are controlled by foot control systems. For example, surgical cutting instruments, endoscopic tools, irrigation and aspiration tools, dental drills and other handpieces, ultrasonic dental scalers, and dental prophylaxis units can be activated with foot control systems. The foot control system includes a footswitch device that is placed on the floor within easy reach of the practitioner. The footswitch is used to activate a dental/medical apparatus, which includes a base-operating unit. A connector cable is used to secure the footswitch to the base unit in a "hard-wired" or "tethered" system. Alternatively, remote, "wireless" foot control systems, which do not use a connector cable, can be used to activate the base unit in some instances. A flexible, instrument cable connects the dental/medical instrument, for example a dental handpiece, to the base unit. The dental or medical practitioner activates the base unit and coupled dental/medical instrument by simply depressing the footswitch with his or her foot. Using such systems allows the practitioner to be "hands-free." The practitioner does not need to manually adjust knobs, dials, and the like on the base unit to control its operational mode. Rather, the practitioner can control the operation of the base unit by using the footswitch.

Some conventional footswitches are referred to as multi-position or multi-staged switches. An operator depresses the pedal of the footswitch to a certain position, and this action causes the dental/medical apparatus to operate in a specific mode. The selected mode of operation is based upon the position of the footswitch pedal. For example, with a two-position footswitch, a dental practitioner can depress the pedal to a first position so that water flows through the handpiece for rinsing the teeth of a patient. Then, the pedal of the footswitch can be depressed to a second position so that a cleaning spray containing anti-microbial medicaments flows through the handpiece for cleaning the teeth.

Various hard-wired and wireless systems, which include a footswitch device for controlling the dental/medical unit, are known in the industry. For example, Warrin et al., U.S. Patents 5,125,837 and 5,419,703 disclose an ultrasonic dental scaler unit having a handpiece and scaling insert that can be used for scaling teeth and providing therapeutic lavage solutions to periodontal pockets in the mouth. The dental scaler unit includes a footswitch device, which is connected to the base unit by an electrical cable. The scaler unit further includes a dental handpiece, which is connected to the base unit by a conduit containing electrical wires and a tube for cooling water. The base unit includes a switch that can be set to a first or second position. The footswitch also can be depressed to a first or second position. The positions of the base unit switch and footswitch make it possible for the practitioner to use the apparatus for scaling only, lavage only, or simultaneous lavage and scaling.

Jovanovic et al., U.S. Patent 5,754,016 describes a hard-wired system that uses a footswitch to control an ultrasonic dental scaler unit. In this system, a cable connects the scaler base unit to the footswitch. The scaler handpiece, which is mounted to the base unit, includes a feedback coil for controlling the amplitude and vibration of the scaling insert, which is placed in the handpiece. The amplitude and frequency of vibration of the scaling insert can be continuously adjusted to maintain constant scaling power. The footswitch device is connected to a boost enabler in the base unit by a connector cable. The footswitch can include first and second electrical switch contact positions, whereby the first switch position provides normal ultrasonic power to the handpiece and the second switch position provides a temporary boost in ultrasonic power.

In Warner, published United States Patent Application US 2004/0115591, a system for remotely controlling multiple medical and dental devices is disclosed. The system includes a wireless handheld unit and foot pedal control unit. The practitioner first presses a button on the handheld unit to transmit a wireless RF signal containing a "device selection message." This signal selects a specific device from the plurality of devices that will be controlled by movement of the foot pedal. For example, the user may select a dental drill from a number of different dental treatment devices. In turn, the user presses the foot pedal and a RF signal containing a "device actuation message" is transmitted to the dental drill; the drill is thus activated.

Takahashi, published United States Patent Application US 2005/0080403 discloses a system for controlling a medical device with a remote control footswitch. The footswitch includes a pedal that is pressed to turn on the medical device. By pressing the pedal, a RF signal is transmitted together with an ID code to the medical device. The medical device, as the control target, receives the transmitted signal, and stores the ID code so as to identify the footswitch. Further, the medical device returns the ID code and, thus, the footswitch stores the ID code so as to identify that particular medical device as the control target. The system described in the '403 application is designed particularly for controlling multiple medical devices in an operatory room with a single footswitch. For example, the footswitch can be used to control an ultrasonic operation apparatus and an electric knife.

Mace, published United States Patent Application US 2005/0147940 discloses a foot control system for dental instruments. The system includes a foot control, which is responsive to actuation by movement of the foot. The foot control generates an electromagnetic control signal that is sent to a receiver, which is connected to the dental instrument. The control signal is received by a receiver circuit that converts the control information into the form required by the dental instrument. The receiver is shown as a separate unit and described as being located in a place that does not interfere with the dental procedure.

While some conventional footswitch systems can be generally effective in controlling dental and medical units there is a need for an improved system. Particularly, it would be desirable to have a footswitch that could be used to activate a dental/ medical unit efficiently in either a hard-wired or wireless system. It also would be advantageous to have a system, whereby the dental/medical unit is capable of responding immediately to a signal transmitted by the footswitch. A system that includes a footswitch and dental/medical unit, each having a unique identification code or address, also would be desirable. With such a system, the footswitch could be reprogrammed to know the identification address of the specific dental/medical unit, assigned thereto. Conversely, the dental/medical unit could be reprogrammed to know the identification address of the specific footswitch assigned thereto. When sending communication signals, if the transmitting component (footswitch or dental/medical unit) did not include both identification addresses, then it would not be able to communicate with the respective receiving component (footswitch or dental/medical unit). This would prevent a footswitch from communicating with the wrong dental/medical unit. The present invention provides such a footswitch system having these objects, features, and advantages as well as others.

### SUMMARY OF THE INVENTION

The present invention relates to a wireless system for controlling a dental or medical treatment apparatus as specified in claim 1. Preferred embodiments are according to the dependent claims. The system includes a footswitch device and a dental/medical base unit, each having a communication element for transmitting and receiving signals. The footswitch device contains first and second switches and has a foot-depressable member, particularly an upper movable cover. Depressing the member to a first position activates the first switch and depressing the member to a second position activates the second switch. The footswitch device further includes a communication element for transmitting a first operational signal in response to the member being depressed to the first position and a second operational signal in response to the member being depressed to the second position.

The dental or medical treatment apparatus includes a communication element for receiving the first and second operational signals from the footswitch. The first signal causes the apparatus to operate in a first mode and the second signal causes the apparatus to operate in a second mode. Different operational modes depending upon the switching signal such as, for example, normal power and boosted ultrasonic power, can be activated in this manner.

The system of this invention includes several improvements over conventional foot control systems. For example, the present system includes an identification synchronization mechanism, wherein the apparatus has an initially programmed "A" identification address and the footswitch has an initially programmed "B" identification address. The apparatus is subsequently reprogrammed to include the "A" and "B" identification addresses and the footswitch is reprogrammed to include the "A" and "B" identification addresses. This reprogramming occurs by a synchronization communication between the apparatus and footswitch. The "A" and "B" identification addresses may be any codes, marks, letters, numbers, or other arbitrary symbols, and any sequences and combinations thereof.

When the system is operating in a wireless mode, the operational signals from the footswitch are transmitted in a wireless manner such as, for example, by radio frequency (RF) signals. Although not forming part of the invention, when the system is operating in a hard-wired mode, the operational signals from the footswitch are transmitted in a hard-wired manner such as, for example, by a connector cable. In the hard-wired mode, wireless communication signals are still transmitted from the footswitch to the apparatus, but the apparatus does not act on these signals. The footswitch continues to send the wireless signals until the apparatus transmits a REQUEST SLEEP signal to the footswitch and the footswitch responds by entering a sleep mode, The wireless and hard-wired systems of this invention are particularly preferred for controlling the operation of an ultrasonic dental scaler.

### BRIEF DESCRIPTION OF THE DRAWINGS

The novel features that are characteristic of the present invention are set forth in the appended claims. However, the preferred embodiments of the invention, together with further objects and attendant advantages, are best understood by reference to the following detailed description in connection with the accompanying drawings in which:
**FIG. 1** is a perspective view of one embodiment of the system of the present invention showing a dental practitioner using a footswitch device to control the operation of an ultrasonic dental scaler apparatus in a remote, wireless system;
**FIG. 2** is a perspective view of one embodiment of the system of the present invention showing a dental practitioner using a footswitch device to control the operation of an ultrasonic dental scaler apparatus in a hard-wired system;
**FIG. 3** is a close-up side perspective view of the footswitch device used in the system shown in FIGS. 1 and 2;
**FIG. 3A** is a bottom perspective view of the footswitch device shown in FIG. 3 with the battery compartment door removed;
**FIG. 4** is a close-up view of one embodiment of an ultrasonic dental scaler apparatus that can be used in the systems shown in FIGS. 1 and 2;
**FIG. 5** is a close-up view of the dental handpiece and scaling insert that can be used with the apparatus shown in FIG. 4;
**FIG. 6** is a close-up view of one embodiment of an ultrasonic dental scaler apparatus that can be used in the systems shown in FIGS. 1 and 2; and
**FIG. 7** is a close-up view of the dental handpiece, scaling insert, and air-polishing insert that can be used with the apparatus shown in FIG. 6.

### DETAILED DESCRIPTION OF THE PREFERRED EMBODIMENTS

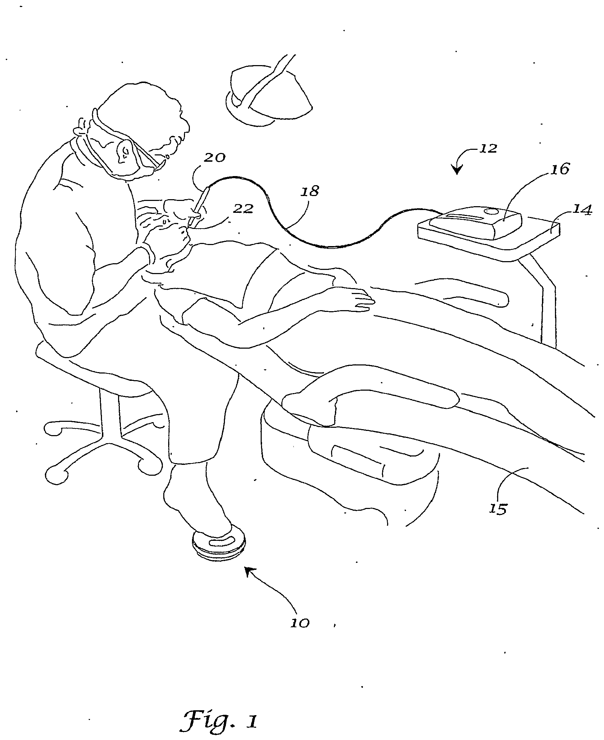

Referring to the drawings, **FIG. 1** illustrates one embodiment of the system of the present invention including footswitch device (10). The footswitch device (10) shown in **FIG. 1** is further described in co-pending, co-assigned patent application entitled "Footswitch for Activating a Dental or Medical Treatment Apparatus," and published as WO2007084605. The footswitch device (10) shown in **FIG. 1** is intended for illustrative purposes only and is not restrictive. It should be understood that other suitable footswitch devices (10) may be used to control the operation of the dental/medical treatment apparatus in accordance with the system of the present invention.

In **FIG. 1****,** a dental practitioner is shown pressing the footswitch device (10) in order to activate a dental treatment apparatus, particularly an ultrasonic dental scaler apparatus (12), which is seated on a tray (14) attached to a dental chair (15). Dental practitioners use ultrasonic dental scalers (12) to provide therapeutic and preventive care to their patients. The ultrasonic scaler (12) is used primarily to remove calculus deposits and heavy plaque from tooth surfaces. The ultrasonic dental scaler (12) includes a power base or drive unit (16). A flexible and lightweight handpiece cable (18) connects a handpiece (20) to the base unit (16). An ultrasonic scaling insert (22) is inserted into the handpiece (20). Different scaling inserts (22) are used depending upon the health of the patient, the tooth to be treated, and the type of calculus/plaque deposits to be removed. The scaling insert (22), which is based on magnetostrictive or piezoelectric technology, vibrates at an ultrasonic frequency to remove deposits from tooth surfaces. In essence, both magnetostrictive and piezoelectric systems convert electric signals into mechanical motion of the scaling insert (22), but they use different mechanisms to do so.

In a piezoelectric system, fixed ceramic crystals in the handpiece (20) vibrate to cause the tip (23) of the scaling insert (22) to move in a linear stroke pattern. In a magnetostrictive system, the handpiece (20) includes an energizing coil that surrounds the scaling insert (22). The scaling insert (22) comprises a transducer that is formed from a stack of laminar plates made of magnetostrictive material. The energizing coil excites the plates of magnetostrictive material via a magnetic field so that the plates longitudinally expand and contract at ultrasonic frequencies. This causes the tip (23) of the scaling insert (22) to vibrate in an elliptical stroke pattern. The tip (23) of the scaling insert (22) vibrates at an ultrasonic frequency, which is defined generally as being within the range of 18 to 50 kHz (18,000 to 50,000 cycles per second). It is common for the scaling insert (22) to have an operational frequency of either 25 kHz or 30 kHz. In addition, the ultrasonic scaling insert (22) typically includes a means for delivering water or other fluid to the tip (23) of the insert (22). The fluid cools the tip (23) and provides other advantages as discussed further below.

Although the footswitch device (10) will be described herein as controlling the operation of an ultrasonic dental scaler (12) primarily, it should be understood that the footswitch (10) can be used to control the operation of any medical or dental treatment apparatus in accordance with the present invention. For example, the footswitch device (10) may be used to control the operation of electrocardiogram machines, X-ray machines, surgical cutting instruments, endoscopic and laproscopic tools, blood analyzers, diagnostic tools, dental chairs, dental irrigators, dental air polishing and prophylaxis systems, dental drills, endodontic and periodontic handpieces, and other dental equipment. The footswitch device (10) is shown in **FIG. 1** as controlling the operation of an ultrasonic dental scaler (12) for illustration purposes only, and **FIG. 1** should not be construed as limiting the scope of the invention.

As shown in **FIG. 1****,** the footswitch device (10) is preferably used to operate a dental/medical treatment apparatus (12) in a wireless, remote control system. The footswitch (10) includes a communication element that transmits a radio frequency (RF) signal to a communication element within the base unit (16) of the dental/medical apparatus (12), which receives the signal. When the communication element in the base unit (16) receives the RF signal, it activates the base unit (16) and the handpiece (20) (or other instrument) coupled to the unit (16). Wireless packets of information including, for example, switch status, addresses, operation frequency, battery status, synchronization modes, identification codes, equipment status, alarm messages, and the like can be sent back and forth between the footswitch device (10) and dental/ medical apparatus (12) using the communication elements of the footswitch (10) and base unit (16) according to this embodiment of the invention. Although it is preferred that RF communication signals be used in the wireless system of this invention, it is recognized that the system could be configured to use other communication signals. For example, it is contemplated that infrared or ultrasound wireless signals could be transmitted and received by the communication elements of the footswitch (10) and base unit (16).

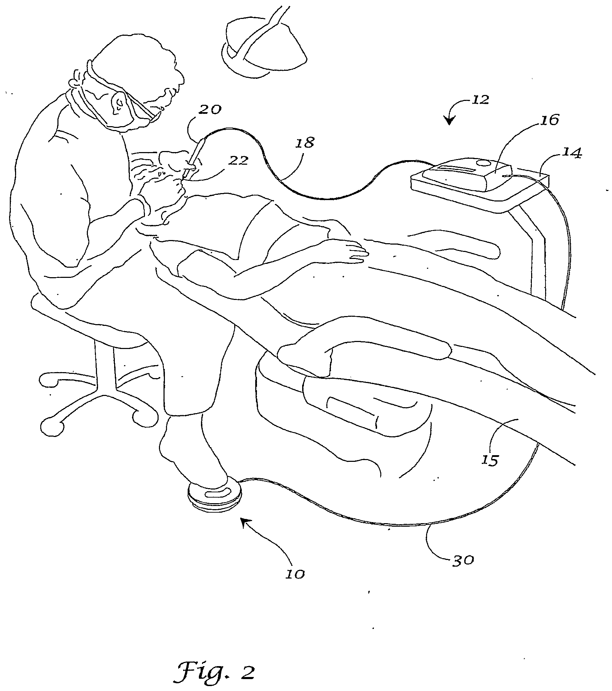

Alternatively, although not forming part of the invention, the footswitch device (10) may be used to operate the dental/medical apparatus (12) in a hard-wired system as shown in **FIG. 2****.** In such a system, the footswitch device (10) is connected to the base unit (16) by a connector cable (30). The footswitch device (10) is tethered to the base unit (16) in this manner. The switching signals are sent back and forth between the footswitch device (10) and base unit (16) via electrical contacts in the connector cable (30). It should be pointed out that in the hard-wired mode, the footswitch (10) still transmits wireless RF communication signals to the base unit (16), but the RF signals are ignored by the base unit (16). In other words, the base unit (16) does not respond to or act on the RF signals. The footswitch (10) will continue to transmit RF signals, until the base unit (16) transmits a "REQUEST SLEEP" signal to the footswitch (10). In response to the "REQUEST SLEEP" signal, the footswitch (10) enters a sleep mode and ceases transmitting RF signals to the base unit (16).

One advantageous feature of the footswitch device (10) is that it can be used in either wireless or hard-wired systems. An auxiliary connector cable (30) can be included with the footswitch (10) in a kit or package, and the cable (30) can be installed to tether the footswitch (10) to the base unit (16) as shown in **FIG. 2****.** This feature is particularly important in the event that the footswitch (10) is unable to communicate with the base unit (16) via wireless signals. For instance, if battery power in the footswitch (10) is too low, the footswitch (10) will not be able to communicate effectively with the base unit (16) via RF signals. Alternatively, there may be sufficient battery power in the footswitch (10), but the practitioner may wish to use the auxiliary connector cable (30) to preserve battery life. In other cases, there may be problems with the communication element or electronics of the footswitch (10) preventing the transmission of wireless signals to the base unit (16). In view of one of the foregoing events, the auxiliary connector cable (30) can be used to connect the footswitch (10) to the base unit (16). Then, the footswitch (10) can communicate with the base unit (16) via switching signals transmitted through the electrical contacts of the connector cable (30). Using the auxiliary connector cable (30) to tether the footswitch (10) to the base unit (16) allows the base unit (16) to continue receiving switching signals from the footswitch (10) in situations when it is not possible to transmit and/or receive wireless switching signals.

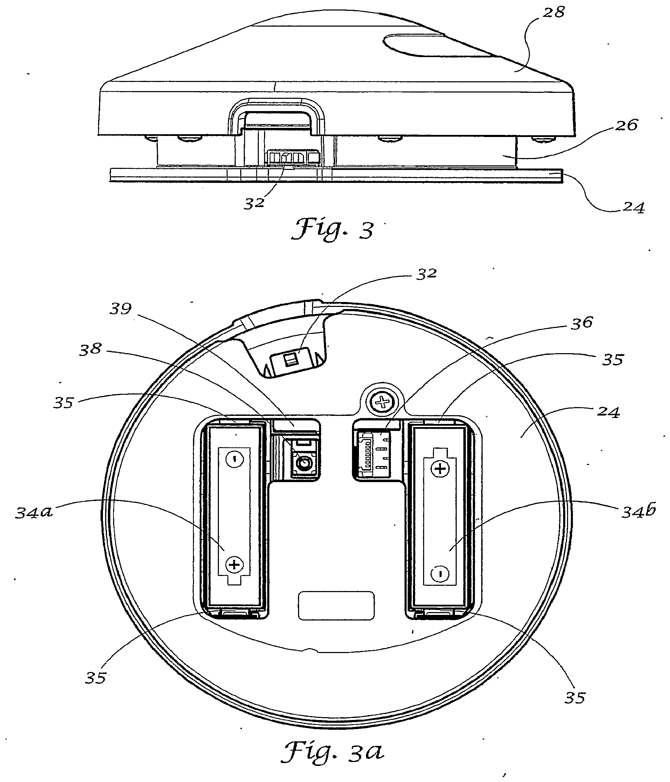

Referring to **FIG. 3****,** a side perspective view of a footswitch device (10), which can be used in the wireless or hard-wired system of this invention, is shown. The footswitch (10) generally includes a base plate (24), a central body or housing (26), and an upper, movable cover (28). In the event that the operator wishes to use a hard-wired system, an auxiliary connector (32) is provided in the footswitch device (10). One end of the connector cable (30) is inserted in the auxiliary connector (32), and the other end of the cable (30) is inserted in the base unit (16) to hard-wire the system. The central housing (26) of the footswitch (10) includes a switching assembly with at least first and second electrical switches.

To activate the footswitch device (10), an operator depresses the spring-biased upper cover (28) to a first position, whereby the first switch contact is closed. One advantageous feature of the footswitch (10) is that the operator may depress any region of the upper cover (28) in order to activate the switching mechanism. For instance, the operator may depress the central region of the upper cover (28). Alternatively, the operator may depress any point along the outer perimeter of the upper cover (28). In other words, the footswitch (10) has a three hundred and sixty-degree (360°) level of perimeter activation. Depressing the upper cover (28) to a first position causes the first switch contact to close. The closing of the first switch contact in the footswitch (10) is presented to a microcontroller located in the footswitch (10) and to a microcontroller located in the base unit (16). A switching signal indicating closure of the first switch contact is transmitted by the connector cable (30) to the base unit (16) (hard-wired systems), or by a RF switching signal sent from the footswitch (10) to the base unit (16) (wireless systems).

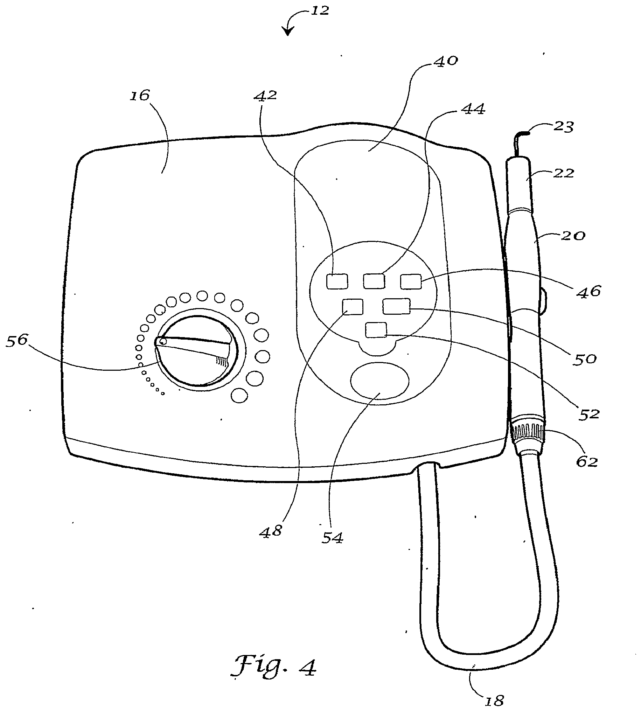

Upon closing the first switch contact, the footswitch (10) is considered to be operating in Stage 1, and the dental handpiece (20) (or other instrument), which is attached to the base unit (16) is powered to run in a first operating mode. In a first embodiment of the ultrasonic dental scaler apparatus (12) (FIG. 4), a dental handpiece (20) containing scaling insert (22) is used to scale tooth surfaces as discussed in further detail below, and Stage 1 is a normal power mode. In other words, normal ultrasonic power is delivered to the dental handpiece (20) when the footswitch (10) is operating in Stage 1. Under normal power, the scaling insert (22) can be used to clean ordinary calculus deposits from tooth surfaces.

An operator can make the footswitch device (10) operate in Stage 2 by exerting additional downward pressure on the upper cover (28). When sufficient force is applied to the upper cover (28), the second switch contact is closed. The closing of the second switch contact is presented to the microcontroller in the base unit (16) by electrical contacts in the connector cable (30) (hard-wired systems) or by a transmitted RF signal (wireless systems). Upon closing the second switch contact, the footswitch (10) is considered to be operating in Stage 2, and the ultrasonic dental handpiece (20) is powered to run in a second mode. In this first embodiment, Stage 2 is a boosted power mode. In other words, a boost in ultrasonic power is delivered to the handpiece (20) when the footswitch device (10) is operating in Stage 2. Under this boosted power, the scaling insert (22) can be used to power away particularly tenacious calculus deposits from tooth surfaces.

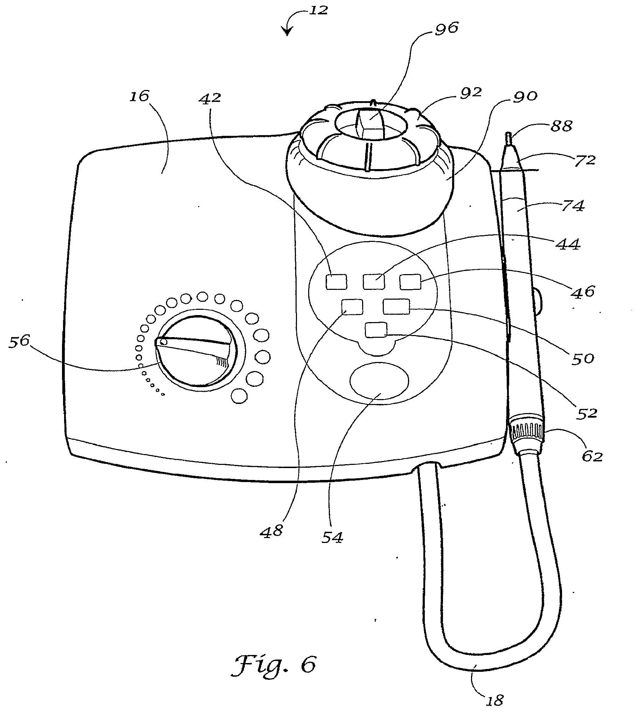

In a second embodiment of the ultrasonic scaler (12) **(****FIG. 6****),** a dental handpiece (70) containing an air polishing insert (72) may be used to clean tooth surfaces as discussed in further detail below. The footswitch (10) may be activated to run in Stage 1 in the same manner as discussed above. But, in this embodiment of the scaler (12), Stage 1 is a lavage (rinse) only operational mode. In this Stage 1 mode, only a rinsing fluid is discharged from the tip (88) of the air polishing insert (72). Upon closing the second switch contact so that the footswitch (10) operates in Stage 2, an air/powder/water slurry is discharged from the tip (88). Stage 2 may be referred to as a powder slurry mode and the slurry may be used to air polish tooth surfaces.

Because of the different levels of pressure that must be applied to the upper cover (28), there is a distinct feel between operating the footswitch device (10) in Stage 1 versus Stage 2. Basically, an operator can depress the upper cover (28) until he or she feels a "click." The footswitch (10) will continue operating in Stage 1 so long as the operator keeps-up the minimum pressure on the upper cover (28). If the operator wishes to operate the footswitch (10) in Stage 2, he or she must apply additional downward pressure on the upper cover (28). In such an event, the operator continues pressing the upper cover (28) downwardly until he or she feels a second "click" indicating that Stage 2 has been activated. The footswitch device (10) will continue operating in Stage 2 so long as the operator maintains sufficient pressure on the upper cover (28). Since Stage 2 requires a different amount of pressure for activation than Stage 1, the operator can distinctly feel when he or she is entering Stage 2. Also, the operator will realize that he or she must maintain this additional pressure to keep the footswitch device (10) running in Stage 2. After using the footswitch (10) over a period of time, the operator will get a "feel" as to the amount of force which must be applied to activate Stage 1 versus the force required for Stage 2.

The different modes of operation of the footswitch (10) and base unit (16) and the communication signals transmitted between the footswitch (10) and base unit (16) are described in further detail below.

### Initial Power Mode

The central housing (26) of the footswitch (10) includes a printed circuit board having electronics that include a microcontroller. The electronics also include a communication element (or transceiver) for transmitting and receiving RF signals. The electronics are coupled to batteries (34A, 34B) which are placed in the battery compartment of the footswitch (10) (FIG. 3A). The batteries (34A, 34B) preferably supply a total of 3 volts to the footswitch (10). Wire leads run from the battery terminals (35) to the circuit board connector (36), which connects the wire leads to the printed circuit board. At initial power or start-up mode, when battery voltage is first applied to the electronics of the footswitch (10), the microcontroller runs through a series of checks. The checks include hardware and software initializations, input/out (I/O) interface checks, random access memory (RAM) checks, and electrical erasable programmable read only memory (EEPROM). Once the footswitch hardware and software have been initialized, the communication element of the footswitch (10) transmits an "AWAKE" signal to the dental scaler base unit (16) at a pre-programmed frequency, which is preferably near 2.4 GHz. The base unit (16), which has the same identification address as the footswitch (10), responds to the "AWAKE" signal by sending an "ACKNOWLEDGMENT" signal and a "REQUEST FOR BATTERY STATUS" signal. These communication signals are sent in a wireless manner, for example, by RF signals, as discussed above. Unique identification addresses are programmed into the base unit (16) and footswitch (10) to ensure that the footswitch is being used with the correct base unit. The synchronization of the base unit address with the footswitch address is discussed in further detail below.

In response to the 'REQUEST FOR BATTERY STATUS" signal received from the base unit (16), the footswitch (10) transmits an "ACKNOWLEDGEMENT" signal to the base unit (16). The footswitch (10) also measures the voltage of the battery, sending this battery data to the base unit (16). If the battery voltage is too low, the Low Battery indicator (42) in the information center (40) of the base unit (16) will light up **(****FIGS. 4** **and** **6****).** If the battery voltage is sufficient, then the system enters a stand-by, ready for operation mode. If there are no other information requests or other signals from the footswitch (10) or base unit (16), which need to be acted upon at this time, the base unit (16) will transmit a "REQUEST SLEEP" signal to the footswitch (10). In response, the footswitch (10) will enter a sleep mode to preserve battery life. The sleep mode of the footswitch (10) is described in further detail below.

### Loaded Battery Test Mode

Since the footswitch (10) and base unit (16) operate with a non-rechargeable battery system, a mechanism for monitoring battery life has been incorporated into the design of the footswitch (10). During boot-up of the microcontroller in the footswitch (10) and upon receiving a "REQUEST FOR BATTERY STATUS" signal from the base unit (16), the microcontroller will apply a load to the batteries (34A, 34B) in the footswitch (10) and measure the voltage of the batteries under the loaded condition. The measured voltage value is digitized and transmitted to the base unit (16), where battery value is evaluated. If the battery voltage is too low, the Low Battery indicator (42) in the information center (40) of the base unit (16) will light up. If the battery voltage is sufficient, then the system will resume a stand-by, ready for operation mode

### Sleep Mode

When the footswitch (10) is in sleep mode, the microcontroller and communication elements of the footswitch (10) show minimal activity. In sleep mode, the electrical current requirements from the batteries (34A, 34B) are significantly reduced, thus extending the life of the batteries. The footswitch (10) enters sleep mode upon one of the following events occurring: 1) a pre-determined period of time has elapsed in which no signals have been received from the base unit (16); 2) no switching events have occurred; or 3) the base unit (16) sends a REQUEST SLEEP signal to the footswitch (10). The footswitch (10) can only exit the sleep mode by a switching event, particularly activating Switch 1, Switch 2, or the Synchronization switch (38) located in the battery compartment of the footswitch (FIG. 3A), or by placing batteries (34A, 34B) in the footswitch (10) and applying battery voltage.

### Synchronization Mode

In order for the footswitch (10) and base unit (16) to properly communicate with each other, the addresses and operating frequencies of the footswitch (10) and base unit (16) must be known to each other. The base unit (16) is programmed initially with an identification address when the printed circuit board of the base unit (16) is manufactured. This initial identification address of the base unit (16) may be arbitrarily referred to as identification address "A." The footswitch (10) also is programmed initially with a default identification address when the printed circuit board of the footswitch is manufactured. This initial identification address of the footswitch (10) may be arbitrarily referred to as identification address "B." Once a footswitch (10) is manufactured and assigned to a base unit (16), the identification address of the footswitch (10) is reprogrammed so that it includes the address of the base unit (16). In other words, the footswitch (10) is reprogrammed to include identification addresses "A" and "B." Each of the "A" and "B" identification addresses may be, for example, a sequence of numbers falling within the range of 00,000,001 to 17,000,000. For example, the base unit (16) may be programmed initially with an "A" identification address of "1,111,111" and the footswitch (10) may be programmed initially with a "B' identification address of "2,222,222." In which case, the footswitch (10) must be reprogrammed so that its address reads " 2,222,222 1,111,111" And, the base unit (16) must be reprogrammed so that its address reads "1,111,111 2,222,222." It should be understood that the foregoing identification addresses A and B are for illustrative purposes only and not meant to be restrictive. Identification addresses A and B can be any codes, marks, letters, numbers, or other arbitrary symbols, and sequences and combinations thereof. The initially programmed identification addresses A and B can be identical, but in most cases these addresses will be different codes, letters, or numbers as illustrated in the above example. This process, referred to as address synchronization of the footswitch (10) and base unit (16), includes the following steps:
(i) the base unit (16) is first powered off by turning off the main power control On/Off switch on the base unit (16);
(ii) a new set of "AA" batteries is placed in the footswitch (10);
(iii) a Purge button (54), located in the information center (40) of the base unit (16) **(****FIGS. 4****,** **6****)** is pressed and held while the power control On/Off switch on the base unit (16) is turned on;
(iv) the Purge button (54) on the base unit (16) is pressed and held until the light-emitting indicators (42, 44, 46, 48, 50, and 52) in the information center (40) begin to blink on and off in sequence - then the Purge button (54) is released. This blinking indicates that the base unit (16) is ready to reprogram the identification address of the footswitch (10). The light-emitting indicators (42, 44, 46, 48, 50, and 52) provide information about the different modes of operation of the scaler apparatus (12) and are discussed in further detail below;
(v) a synchronization button (38) located on the footswitch (10) is pressed **(****FIG. 3A****).** The synchronization button (38) is normally located in the battery compartment of the footswitch (10) and maybe color-coded for ease of identification. For example, the synchronization button may be red-colored. At this point a light-emitting diode (LED) (39) located near the synchronization button (38) of the footswitch (10) begins to blink on and off. In turn, the indicators (42, 44, 46, 48, 50, and 52) in the information center (40) begin to blink simultaneously.

This blinking indicates that the footswitch (10) and base unit (16) are communicating with each other in a wireless manner. The footswitch identification address is being reprogrammed to include the originally programmed footswitch address (for example, 2,222,222) as well as the base unit address (for example, 1,111,111). At the same time, the base unit identification address is being reprogrammed to include the originally programmed base unit address (for example, 1,111,111) as well as the footswitch address (for example, 2,222,222). When the footswitch (10) and base unit (16) stop blinking this means that reprogramming has been completed and the identification addresses of the footswitch (10) and base unit (16) have been synchronized. The footswitch (10) has communicated its initially programmed identification address to the base unit (16), and the base unit (16) has communicated its initially programmed identification address to the footswitch (10). In the above example, upon synchronization, the identification address of the footswitch (10) will read as the numerical sequence: "2,222,222 1,111,111" and the identification address of the base unit (16) will read as the numerical sequence: "1,111,111 2,222,222."

This initial, start-up synchronization communication between the base unit (16) and footswitch (10) is an important mechanism of the system of the present invention. The synchronization of the addresses occurs during a first wireless communication between the base unit (16) and footswitch (10) prior to any other communication signals. Both identification addresses are included in subsequent communications between the footswitch (10) and base unit (16). In other words, communication signals transmitted from the footswitch (10) to the base unit (16) include identification addresses "A" and "B," and communication signals transmitted from the base unit (16) to the footswitch (10) also include identification addresses "A" and "B." Once the identification addresses are reprogrammed so that the footswitch (10) knows the address of the base unit (16) and the base unit (16) knows the address of the footswitch (10), the footswitch (10) and base unit (16) can communicate with each other. If the footswitch (10) does not know the identification address of the base unit (16) and the base unit (16) does not know the identification address of the footswitch (10), then the footswitch (10) and base unit (16) will not be able to communicate with each other. This identification mechanism prevents the footswitch (10) from communicating with the wrong dental/medical apparatus (12). For example, if there are multiple dental apparatus (12) and footswitches (10) in a dental operatory room, there maybe some confusion as to which footswitch controls which apparatus. One footswitch may control a dental chair, where the patient sits, while a second footswitch may control an X-ray camera. Sill another footswitch may control an ultrasonic dental scaler/cleaning apparatus. The dentist will want to exercise care in selecting and using a footswitch so that he or she does not activate the wrong apparatus. The identification mechanism of this invention acts as an operational check and prevents the wrong apparatus from being activated in error.

### Normal Operational Mode

Upon activating Switch 1 or Switch 2 of the footswitch device (10), the communication element (transceiver) in the electronics of the footswitch (10) transmits an "AWAKE" signal and a "SWITCH POSITION STATUS" signal (indicating whether Switch 1 or Switch 2 has been activated) to the base unit (16). The communication element in the electronics of the base unit (16) receives the signals and the unit operates accordingly. For example, as discussed above, activating Switch 1 can cause the base unit (16) to operate in a normal ultrasonic power mode, while activating Switch 2 can cause the base unit (16) to operate in a boosted ultrasonic power mode. Once Switch 1 or Switch 2 has been activated and footswitch (10) is considered turned "On," the base unit (16) transmits a "REQUEST FOR SWITCH POSITION STATUS" signal at pre-determined time intervals, and the footswitch (10) responds by sending a "SWITCH POSITION STATUS" signal to the base unit (16). Sending and receiving these signals ensures that the footswitch (10) and base unit (16) are in constant synchronization with each other. This "handshaking" communication means that the base unit (16) can respond instantaneously to any change in the position of Switch 1 or 2. Because of these communication signals, the base unit (16) will respond instantaneously to the closing or opening of Switch 1 or 2 in the footswitch (10). Thus, the dental practitioner can precisely and directly control the operation of the base unit (16) and instruments by activating the footswitch device (10). The practitioner can keep his or her hands free while working with the footswitch device (10) and is better able to concentrate on performing the dental procedure.

When the system is hard-wired, the footswitch (10) transmits the above-described wireless communication signals to the base unit (16), but the wireless signals are ignored by the base unit (16). The base unit (16) does not respond to or act upon the wireless signals transmitted by the footswitch (10) in this mode, because the base unit is receiving the switching signals via electrical contacts in the connector cable (30) as discussed above. Eventually, the base unit (16) transmits a "REQUEST SLEEP" signal to the footswitch (10) communicating to the footswitch (10) that there is no reason to continue sending wireless signals, and the footswitch should enter a sleep mode to preserve battery life. In response to the "REQUEST SLEEP" signal, the footswitch (10) enters a sleep mode and stops transmitting RF signals to the base unit (16). The dentist activates the first and second switches by deftly depressing the upper cover (28) of the footswitch device (10), as discussed above, and the base unit (16) responds immediately. Preferably, the footswitch sends a "SWITCH POSITION STATUS" signal every 250 milliseconds (ms) to ensure precise coordination between the footswitch (10) and base unit (16). Of course, the "SWITCH POSITION STATUS" signal can be programmed so that it is transmitted at a different pre-determined timing interval (for example, every 800 milliseconds) if such a signal is desired. Each switching event (closing or opening of the first or second switch) in the footswitch (10) generates an interrupt to the 250 ms timed (or other pre-determined timing interval) transmissions of the "SWITCH POSITION STATUS" signals. The base unit (16) receives this interrupt signal and responds immediately to the switching event.

If the footswitch. (10) sends the signal, "ALL SWITCHES INACTIVE," the base unit (16) will respond by sending a "SLEEP REQUEST" signal instructing the footswitch (10) to go to sleep to preserve battery life. If the footswitch (10) is sending "SWITCH POSITION STATUS" signals such as Switch (Position) 1 ACTIVE signals or Switch (Position) 2 ACTIVE signals to the base unit (16), and the footswitch (10) ceases sending such "SWITCH POSITION STATUS" signals without first sending an "ALL SWITCHES INACTIVE" signal, the base unit (16) shall wait for a pre-determined "time-out" period such as, for example, 750 milliseconds in hope of receiving a response from the footswitch (10). If no response is received during the "time-out" period, then the current mode of operation such as, for example, normal ultrasonic power mode (Switch 1 active) or boosted ultrasonic power mode (Switch 2 active) is disabled.

### Watch Dog Back-Up Mode

A back-up feature is preferably incorporated into the communication system between the footswitch (10) and base unit (16). This back-up feature significantly reduces any chance that the system will remain constantly powered on if communication between the footswitch (10) and base unit (16) is lost for some reason. When operating in the normal mode, the footswitch (10) continues to transmit switch status information at the pre-determined timing interval, for example, every 250 ms. The base unit (16) anticipates this switch status update and operates a countdown timer. If three or more switch status updates are missed in sequence, the base unit (16) will immediately disable the ultrasonic power to the handpiece (20) and attempt to reestablish communication with the footswitch (10).

### Frequency Change Mode

The footswitch (10) and base unit (16) system operates on a frequency selected from sixteen possible channels having frequencies ranging from 2405 KHz to 2480 KHz. Channel to channel separation is 5 KHz. The base unit (16) constantly monitors the selected frequency channel. A RF power time averaging algorithm is incorporated into the base unit (16). When an erroneous power level is detected above a pre-determined threshold, the base unit (16) sends a "REQUEST FOR CHANNEL CHANGE" signal to the footswitch (10). The footswitch (10) searches the remaining frequency channels in sequence for a power level detection below the acceptable threshold. Once a clear frequency channel has been located, both the footswitch (10) and base unit (16) change to the new channel and reestablish communication. The changing of the frequency channels is undetectable by the practitioner operating the system.

### Scan Mode

If the footswitch (10) transmits a signal and does not receive an acknowledgement signal from the base unit (16) in a given time period, it will search each frequency channel looking-for the base unit (16) with the correct address. If the base unit (16) is located at a different frequency channel, the new frequency channel is loaded into the system memory and operation resumes at the correct frequency channel.

Referring to **FIG. 4****,** one possible embodiment of an ultrasonic dental scaler apparatus (12), which can be used in the wireless or hard-wired system of the present invention, is shown in detail. A standard power cord (not shown) connects the base unit (16) to an electrical outlet that supplies power (100-240 volts). One end of the power cord is inserted into a power-input connector located on the backside panel of the unit (16), and the other end is plugged into a standard AC wall outlet. A water supply line (not shown), which may include a water filter, is used to provide water and other lavage fluids to the dental scaler system. One end of the water supply line is inserted into a connector located on the backside panel of the base unit (16). The other end of the water supply line is connected to a dental office water line or a fluid-dispensing device. With a fluid dispensing device, the dental practitioner can select either water or medicament fluids for delivery to the dental scaler system. A main power control On/Off switch is located on the underside panel of the base unit (16).

The base unit (16) encloses a printed circuit board (not shown) having electronics that includes a microcontroller. The electronics also include a communication element (or transceiver) for transmitting and receiving RF signals. An information center (40) is located on the topside panel (face) of the base unit (16). The information center includes various graphic indicators (42, 44, 46, 48, 50, and 52) that light-up to indicate a mode of operation of the dental scaler system as well as a push button light-up indicator (54) for purging the scaler system.

The graphic light-emitting indicators (42, 44, 46, 48, 50, and 52) provide information about which mode of operation the dental scaler system is functioning therein at a given moment. In the base unit (16) shown in **FIG. 4****,** the indicators provide information on low battery power in footswitch (10); service required; power boost; blue zone; rinse mode; and 24 volt power status. It is understood that the indicators (42, 44, 46, 48, 50, and 52) illustrated in **FIG. 4** represent only some examples of the many possible indicators that can be employed. Different indicators may be included in the information center (40) if desired. In the illustrated embodiment, the Low Battery indicator (42) illuminates when the batteries (34A, 34B) in the footswitch (10) are approaching end of life. It is recommended that the batteries (34A, 34B) be replaced at this point.

The Blue Zone indicator (44) provides information about a type of scaling procedure. One area of a patient's dental anatomy where heavy plaque and calculus tend to accumulate is on the surface of the tooth lying below the crest of the gingival tissue (sub-gingival area), and this area should be cleaned thoroughly. The sub-gingival area, however, is particularly sensitive. Gingival fluid and blood often accumulate in the sub-gingival area as the area is cleaned with hand instruments. Patients often feel very uncomfortable during this cleaning process. Dental practitioners face the difficult task of cleaning the sub-gingival area thoroughly while keeping the patient comfortable. To overcome such difficulties, the base unit (16) can be operated at low ultrasonic power for a prolonged time period. This allows the practitioner to clean the sub-gingival area with high clinical efficiency while the patient remains comfortable. When the base unit (16) is operating under these conditions, it is referred to as operating in the blue zone, and the Blue Zone indicator (44) in the information center (40) lights-up accordingly.

Concerning the Power Boost indicator (46), when a temporary boost in ultrasonic power is needed for the scaler apparatus (12), the practitioner can further depress the upper cover (28) of the footswitch (10) to a second position. This action causes a second switch in the footswitch (10) to be activated. The footswitch (10) enters Stage 2, and there is a temporary increase in the ultrasonic power output of the base unit (16). The Power Boost indicator (46) in the information center (40) is illuminated when the base unit (16) is operating at boosted power.

The Service Required indicator (48) lights-up when the system is not functioning properly. The Service Required indicator (48) may be programmed to illuminate in different ways to signify different problems with the system. For example, the Service Required indicator (48) may blink on and off when the system is not operating according to factory specifications. The Service Required indicator (48) may blink at a faster or slower rate if the handpiece (20) status is incorrect or missing from the base unit (16). On the other hand, the Service Required indicator (48) may emit a steady light if the system is overheating.

The Rinse indicator (50) lights-up when the scaler system is operating in a rinse mode. Particularly, the scaling insert (22) includes a means for delivering a rinsing fluid, such as tap water, to the tip (23) of the insert (22). As discussed above, a water supply line connects the base unit (16) to a fluid dispensing system in the dental office. The handpiece connector cable (18) includes a conduit for transporting the fluid to the handpiece (20) and scaling insert (22). The water or other rinsing fluid can be used to irrigate the area in the oral cavity, where the dental work is being preformed, and clean the area of debris. When the scaler system is operating in the rinse mode, the Rinse indicator (50) in the information center (40) is illuminated. Turning the ultrasonic power adjustment knob (56), located on the topside panel, in a full counter-clockwise direction can activate the rinse mode of operation. The practitioner turns the power adjustment knob (56) counter-clockwise until he or she feels a "click," to initiate the rinsing step.

Lastly, the On/Off (24 volt power status) indicator (52) is illuminated when the main power control on/off switch, located on the underside panel of the base unit (16), rests in an "On" position.

It may be desirable to purge the lines of the dental scaler system with tap water at various times, for example, when starting-up the system at the beginning of the day. In order to purge the system, the handpiece (20), without the scaling insert (22) positioned therein, is held over a sink and the Purge button (54) on the base unit (16) is depressed. During the purging step, water is flushed through the lines of the system for a given period of time, for example, two minutes. The Purge button (54) is illuminated when the purge function is activated.

An ultrasonic power adjustment knob (56) is located on the topside panel of the base unit (16). The amount of ultrasonic power, which is transmitted to the scaling insert (22), can be finely tuned by turning the power adjustment knob (56). As discussed above, ultrasonic power is used to generate movement of the scaling insert (22). Increasing the ultrasonic power increases the distance that the tip (23) of the scaling insert (22) moves without changing the frequency of tip movement. The base unit (16) may include a scale (57) with a series of graphic symbols at fixed points indicating the relative level of ultrasonic power being transmitted to the scaling insert (22). The scale (57) is printed on the topside panel of the base unit (16) in a semi-circular pattern around the power adjustment knob (56). The practitioner can manually turn the knob (56) so that it points to a symbol on the scale (57). By turning the knob (56) in this manner, the practitioner can finely tune the level of ultrasonic power depending upon the procedure and needs of the patient. This "hands-on" feature can be used as an alternative to the footswitch (10) for adjusting the ultrasonic power from normal to boost. The lower one-third portion of the scale (57) indicates a relatively low level of ultrasonic power and is considered the Blue Zone. When the power adjustment knob (56) is turned so that it points to this area, the Blue Zone indicator (54) will illuminate.

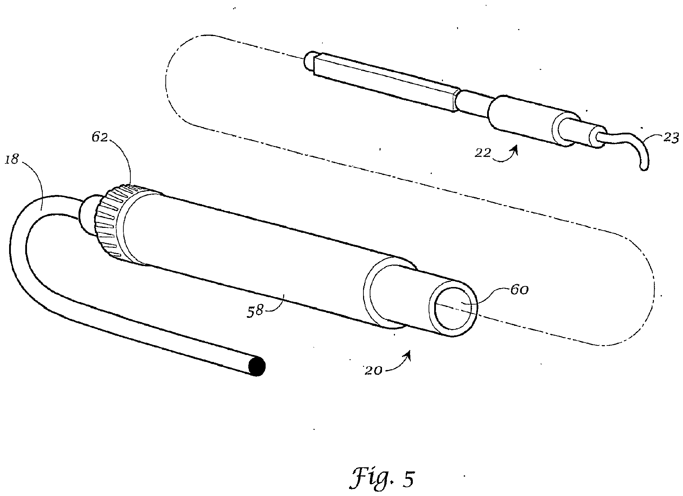

Referring now to **FIG. 5****,** the handpiece (20) and ultrasonic scaling insert (22) used in the scaler (12) of **FIG. 4** are shown in more detail. The handpiece (20) includes a housing (58) with an insert port (60) for placing the scaling insert (22) therein.

In addition, the handpiece (20) includes a lavage control knob (62) that can be rotated for adjusting the flow of lavage fluid through the handpiece (20) and to the scaling insert (22). Tap water normally is used as the lavage fluid. It is important that cooling fluids such as, for example, water, be delivered to the tip (23) of the scaling insert (22) for several reasons.

First, as the tip (23) of the scaling insert (22) makes contact with the tooth and ultrasonically vibrates, heat is generated at the surface of the tooth. The patient may experience a painful sensation if excessive pressure is applied to the scaling insert (22) as the tooth is being cleaned. The cooling fluid, which is supplied to the tip (23) of the scaling insert (22), removes heat from the tooth surface and helps to minimize pain. Secondly, the cooling fluid can be used to irrigate the working area in the oral cavity and clean the area of debris. Thirdly, the magnetostrictive heating element of the scaling insert (22) generates internal heat due to vibration of the laminar stack of magnetostrictive material. The cooling fluid may first be circulated around the transducer to cool the laminar stack. The internal heat is dissipated by means of the cooling fluid as it flows over the laminar stack.

Turning the lavage flow control knob in a clockwise direction increases the flow of fluid at the insert tip (23). While, turning the lavage flow control knob in a counter-clockwise direction decreases the flow of fluid at the insert tip (23). The flow rate of fluid through the handpiece (20) and to the insert tip (23) also determines the temperature of the fluid. In general, fluid that flows at a relatively high rate through the handpiece (20) has a cooler temperature than fluid that flows at a relatively low rate.

**FIG. 6** shows a second possible embodiment of an ultrasonic dental scaler apparatus (12) that can be used in the wireless or hard-wired system of the present invention. The base unit (16) shown in **FIG. 6** can be used to perform ultrasonic scaling and air polishing cleaning procedures. The scaling/cleaning system shown in **FIG. 6** includes many similar components to the ultrasonic scaling system shown in **FIG. 4****,** and like reference numerals are used to identify like components. As discussed above, ultrasonic scaling is used normally to remove calculus deposits and heavy plaque from the tooth surfaces. Ultrasonic scaling procedures also may be used for periodontal debridement in treating periodontal diseases. On the other hand, air polishing is a prophylaxis procedure used to remove extrinsic stains from tooth surfaces such as, for example, stains caused by tobacco, coffee, and tea. Air polishing also may be used to remove soft debris and prepare the tooth surfaces for bonding and sealants.

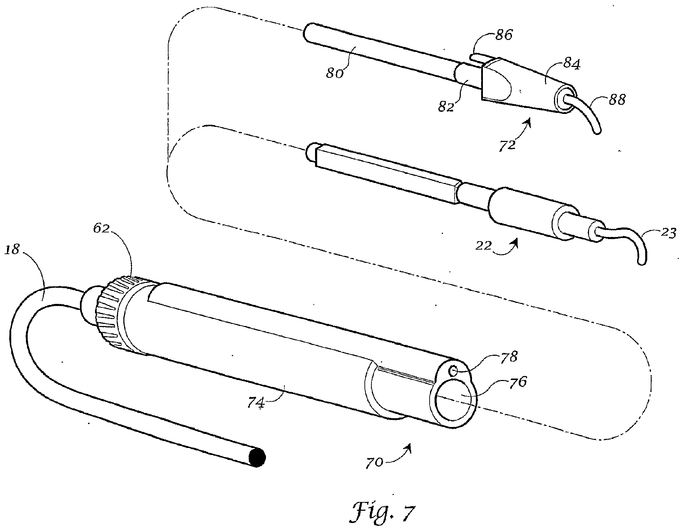

As shown in **FIG. 7****,** a single handpiece (70) can be used to perform both treatment procedures, but different dental inserts (22, 72) are placed in the handpiece (70) depending upon the procedure to be performed. The handpiece (70) includes a housing (74) with an insert port (76) for placing a selected insert (22, 72) therein, and a powder delivery port (78) for delivering powder slurry. When a dental practitioner wishes to operate the system in an ultrasonic scaling mode, he or she places the ultrasonic scaling insert (22) into the handpiece (70). The insert (22) is gently pushed-twisted into the handpiece (70) until it is fully seated. Now, the practitioner can use the handpiece (70) with insert (22) to ultrasonically clean the teeth of a patient in the same manner as the above-described handpiece (20) is used.

If the practitioner wishes to polish the patient's teeth, he or she removes the scaling insert (22) and places an air polishing insert (72) in the handpiece (70). The air polishing insert (72) delivers an air/powder/water slurry to polish tooth surfaces. Air and water pressure is used to deliver a controlled stream of cleaning powder through the handpiece (70). Preferably, a sodium bicarbonate powder composition is used. The sodium bicarbonate powder is watersoluble and leaves no gritty residue on the tooth surfaces. The air polishing insert (72) includes a heating element (80) for heating water that passes over the element (80). A water inlet (82) is provided which permits the water to pass over the heating element (80) and into a nozzle (84). A powder inlet tube (86) is adapted to fit into the powder delivery port (78) of the handpiece (70). Air and powder are directed through the powder inlet tube (86) to the insert tip (88). Water is directed from the nozzle (84) to the insert tip. Then, the air/powder/water slurry is discharged at the insert tip (88) to the targeted area.

Referring back to **FIG. 6****,** the chamber (90) in the base unit (16) that stores and dispenses the cleaning powder is shown. If the practitioner wishes to use the cleaning powder, he or she unscrews the powder fill cap (92), pours the powder into the chamber (90), and screws the cap (92) back onto the chamber. The center of the fill cap (92) includes a cap pointer (94), which is integrally molded to a transparent T-shaped cap handle (96). The flow rate of the powder can be adjusted by rotating the cap pointer (94). Turning the pointer to the "H" symbol (12:00 position) on the cover plate of the chamber (90) will deliver the powder slurry at a rate needed for removing heavy stain. Turning the pointer to the "L" symbol (6:00 position) will deliver the powder slurry at a rate suitable for removing light stain. The pointer (94) can be set at any position between H and L by turning it in either a clockwise or counterclockwise direction. For instance, the pointer (94) can be turned to the "M" position for delivering powder at a rate suitable for removing medium stain. The transparent handle (96) at the center of the fill cap (92) allows the practitioner to see the powder fluffing in the chamber (90) and flowing to the handpiece (70) when the powder dispensing system is operating.

Detailed embodiments of ultrasonic dental scaler apparatus (12) and coupled handpieces (20, 72) are described above and shown in **FIGS. 4-7****,** but it should be understood that these embodiments are illustrative only and not meant to restrict the invention. Other dental and medical treatment apparatus may be used in the wireless and hard-wired systems of the invention.

Workers skilled in the art will appreciate that various modifications can be made to the illustrated embodiments and description herein without departing from the spirit and scope of the present invention. For example, the footswitch device (10) of this invention could contain more than two switches. More particularly, as one example, the footswitch device (10) could contain three switches. Upon activating the first switch, the footswitch would run in Stage 1. Activating the second switch would cause the footswitch to run in Stage 2, and activating the third switch would cause the footswitch to run in Stage 3. In Stage 1, the dental/medical apparatus could operate under normal power. In Stage 2, the dental/medical apparatus could operate under intermediate power, and in Stage 3, the dental/medical apparatus could run under high power. In another embodiment, the footswitch (10) could include a rheostat to sense the downward position of the upper cover (28) of the footswitch (10), thus allowing the operator to have seemingly analog control of a mode of operation on the dental/medical apparatus. This operational mode could be ultrasonic power control or possibly others.

It is also recognized that the dental/medical apparatus may have different modes of operation, and the footswitch can be used to control these different modes. In other words, the footswitch can be used to activate operations other than normal ultrasonic power/boosted power or rinse only/cleaning powder slurry. For example, in the case of an electrical surgical knife, when Stage 1 is activated, the knife may be programmed to operate in a cutting mode only. Then, upon activating Stage 2, the knife may run simultaneously in a cutting mode and lavage mode. In the lavage mode, antibacterial solutions could be dispensed into the surgical area.

The foregoing are only some examples of modifications that can be made to the illustrated embodiments and description herein without departing from the scope of the appended claims.

## Claims

1. A system for controlling a dental or medical treatment apparatus, comprising:
(i) a footswitch device containing a first switch and second switch and having a foot-depressable member, the member being depressed to a first position for activating the first switch and the member being depressed to a second position for activating the second switch, the device further including a communication element for transmitting a first operational signal in a wireless manner in response to the member being depressed to the first position and a second operational signal in a wireless manner in response to the member being depressed to the second position; and
(ii) a dental or medical treatment apparatus including a communication element for receiving the first and second operational signals from the footswitch, the first signal causing the apparatus to operate in a first mode and the second signal causing the apparatus to operate in a second mode, the apparatus having an initially programmed "A" identification address and the footswitch having an initially programmed "B" identification address,
wherein the apparatus is reprogrammed to include said "A" and "B" identification addresses and the footswitch is reprogrammed to include said "A" and "B" identification addresses by a synchronization communication between the apparatus and footswitch; and
wherein the apparatus periodically transmits a "REQUEST FOR SWITCH POSITION STATUS" signal to the footswitch and the footswitch responds by transmitting a "SWITCH POSITION STATUS" signal to the apparatus.

2. The system of claim 1, wherein the system is used to control the operation of a dental treatment apparatus.

3. The system of claim 2, wherein the dental treatment apparatus is an ultrasonic dental scaler.

4. The system of claim 3, wherein activating the first switch causes the dental scaler to operate at normal ultrasonic scaling power.

5. The system of claim 3, wherein activating the second switch causes the dental scaler to operate at boosted ultrasonic scaling power.

6. The system of claim 1, wherein the system is used to control the operation of a medical treatment apparatus.

7. The system of claim 1, wherein the footswitch transmits an "AWAKE" signal and "SWITCH POSITION STATUS" signal to the apparatus upon activation of the first switch in the footswitch, and the apparatus responds to the signals by operating in a first mode.

8. The system of claim 1, wherein the footswitch transmits an "AWAKE" signal and "SWITCH POSITION STATUS" signal to the apparatus upon activation of the second switch in the footswitch, and the apparatus responds to the signals by operating in a second mode.

9. The system of claim 1, wherein the apparatus transmits a "REQUEST FOR SWITCH POSITION STATUS" signal to the footswitch at time intervals of 250 milliseconds.

10. The system of claim 1, wherein the footswitch is powered by battery.

11. The system of claim 10, wherein the footswitch transmits an "AWAKE" signal and the base unit responds by transmitting an "ACKNOWLEDGMENT" signal and "REQUEST FOR BATTERY STATUS" signal to the footswitch.

12. The system of claim 11, wherein the footswitch responds to the REQUEST FOR BATTERY STATUS" signal by transmitting an "ACKNOWLEDGMENT" signal and battery voltage data to the apparatus.

13. The system according to claim 1, whereby the footswitch transmits operational signals in a wireless manner to the dental or medical treatment apparatus, until the apparatus transmits a REQUEST SLEEP signal to the footswitch and the footswitch responds by entering a sleep mode, the wireless operational signals being ignored by the apparatus.

14. The system of claim 13, wherein the system is used to control the operation of a dental treatment apparatus.

15. The system of claim 14, wherein the dental treatment apparatus is an ultrasonic dental scaler.

## Patentansprüche

1. System zum Steuern einer zahnärztlichen oder medizinischen Behandlungsvorrichtung, umfassend:
(i) eine Fußschaltervorrichtung, die einen ersten Schalter und einen zweiten Schalter enthält und ein mit dem Fuß drückbares Element aufweist, wobei das Element zum Aktivieren des ersten Schalters in eine erste Stellung niedergedrückt wird und das Element zum Aktivieren des zweiten Schalters in eine zweite Stellung niedergedrückt wird und die Vorrichtung ferner ein Kommunikationselement umfasst zum Senden eines ersten Betriebssignals in einer drahtlosen Weise als Reaktion darauf, dass das Element in die erste Stellung niedergedrückt wird, und zum Senden eines zweiten Betriebssignals in einer drahtlosen Weise als Reaktion darauf, dass das Element in die zweite Stellung niedergedrückt wird; und
(ii) eine zahnärztliche oder medizinische Behandlungsvorrichtung, die ein Kommunikationselement zum Empfangen der ersten und zweiten Betriebssignale von dem Fußschalter umfasst, wobei das erste Signal die Vorrichtung veranlasst, in einem ersten Modus zu arbeiten, und das zweite Signal die Vorrichtung veranlasst, in einem zweiten Modus zu arbeiten, und die Vorrichtung eine anfänglich programmierte Identifikationsadresse "A" und der Fußschalter eine anfänglich programmierte Identifikationsadresse "B" aufweist;
wobei die Vorrichtung neu programmiert wird, sodass sie die Identifikationsadressen "A" und "B" umfasst und der Fußschalter durch eine Synchronisationskommunikation zwischen der Vorrichtung und dem Fußschalter neu programmiert wird, sodass er die Identifikationsadressen "A" und "B" umfasst; und
wobei die Vorrichtung periodisch ein Signal "ANFORDERUNG SCHALTSTELLUNGSSTATUS" an den Fußschalter sendet und der Fußschalter durch Senden eines Signals "SCHALTSTELLUNGSSTATUS" an die Vorrichtung antwortet.

2. System nach Anspruch 1, wobei das System verwendet wird, um den Betrieb einer zahnärztlichen Behandlungsvorrichtung zu steuern.

3. System nach Anspruch 2, wobei die zahnärztliche Behandlungsvorrichtung ein Ultraschallzahnsteinentferner ist.

4. System nach Anspruch 3, wobei das Aktivieren des ersten Schalters den Zahnsteinentferner veranlasst, bei normaler Ultraschallzahnsteinentfernungsleistung zu arbeiten.

5. System nach Anspruch 3, wobei das Aktivieren des zweiten Schalters den Zahnsteinentferner veranlasst, bei erhöhter Ultraschallzahnsteinentfernungsleitung zu arbeiten.

6. System nach Anspruch 1, wobei das System verwendet wird, um den Betrieb einer medizinischen Behandlungsvorrichtung zu steuern.

7. System nach Anspruch 1, wobei der Fußschalter nach Aktivieren des ersten Schalters in dem Fußschalter ein "AUFWECK"-Signal und ein "SCHALTSTELLUNGSSTATUS"-Signal an die Vorrichtung sendet und die Vorrichtung auf die Signale durch Arbeiten in einem ersten Modus anspricht.

8. System nach Anspruch 1, wobei der Fußschalter nach Aktivieren des zweiten Schalters in dem Fußschalter ein "AUFWECK"-Signal und ein "SCHALTSTELLUNGSSTATUS"-Signal an die Vorrichtung sendet und die Vorrichtung auf die Signale durch Arbeiten in einem zweiten Modus anspricht.

9. System nach Anspruch 1, wobei die Vorrichtung ein Signal "ANFORDERUNG SCHALTSTELLUNGSSTATUS" in Zeitintervallen von 250 Millisekunden an den Fußschalter sendet.

10. System nach Anspruch 1, wobei der Fußschalter mittels Batterie betrieben wird.

11. System nach Anspruch 10, wobei der Fußschalter ein "AUFWECK"-Signal sendet und die Basiseinheit durch Senden eines "BESTÄTIGUNGS"-Signals und eines Signals "ANFORDERUNG BATTERIESTATUS" an den Fußschalter antwortet.

12. System nach Anspruch 11, wobei der Fußschalter auf das Signal "ANFORDERUNG BATTERIESTATUS" durch Senden eines "BESTÄTIGUNGS"-Signals und von Batteriespannungsdaten an die Vorrichtung antwortet.

13. System nach Anspruch 1, wobei der Fußschalter Betriebssignale in einer drahtlosen Weise an die zahnärztliche oder medizinische Behandlungsvorrichtung sendet, bis die Vorrichtung ein Signal "ANFORDERUNG SCHLAF" an den Fußschalter sendet und der Fußschalter durch Eintreten in einen Schlafmodus reagiert, wobei die drahtlosen Betriebssignale von der Vorrichtung ignoriert werden.

14. System nach Anspruch 13, wobei das System verwendet wird, um den Betrieb einer zahnärztlichen Behandlungsvorrichtung zu steuern.

15. System nach Anspruch 14, wobei die zahnärztliche Behandlungsvorrichtung ein Ultraschallzahnsteinentferner ist.

## Revendications

1. Système de commande d'un appareil de traitement dentaire ou médical comprenant :
(i) un dispositif interrupteur au pied contenant un premier interrupteur et un second interrupteur et ayant un élément pouvant être pressé par le pied, l'élément étant pressé dans une première position pour activer le premier interrupteur et l'élément étant pressé dans une seconde position pour activer le second interrupteur, le dispositif incluant en outre un élément de communication pour transmettre un premier signal opérationnel de manière sans fil en réponse à la pression de l'élément dans la première position et un second signal opérationnel de manière sans fil en réponse à la pression de l'élément dans la seconde position ; et
(ii) un appareil de traitement dentaire ou médical incluant un élément de communication pour recevoir les premier et second signaux fonctionnels de l'interrupteur au pied, le premier signal provoquant le fonctionnement de l'appareil dans un premier mode et le second signal provoquant le fonctionnement de l'appareil dans un second mode, l'appareil ayant une adresse d'identification « A » initialement programmée et l'interrupteur au pied ayant une adresse d'identification « B » initialement programmée,
dans lequel l'appareil est reprogrammé pour inclure lesdites adresses d'identification « A » et « B » et l'interrupteur au pied est reprogrammé pour inclure lesdites adresses d'identification « A » et « B » par une communication de synchronisation entre l'appareil et l'interrupteur au pied ; et
dans lequel l'appareil transmet périodiquement un signal « DEMANDE D'ÉTAT DE POSITION D'INTERRUPTEUR » à l'interrupteur au pied et l'interrupteur au pied répond en transmettant un signal « ÉTAT DE POSITION D'INTERRUPTEUR » à l'appareil.

2. Système selon la revendication 1, dans lequel le système est utilisé pour contrôler le fonctionnement d'un appareil de traitement dentaire.

3. Système selon la revendication 2, dans lequel l'appareil de traitement dentaire est un détartreur dentaire à ultrasons.

4. Système selon la revendication 3, dans lequel l'activation du premier interrupteur entraine le fonctionnement du détartreur dentaire à une puissance de détartrage ultrasonique normale.

5. Système selon la revendication 3, dans lequel l'activation du second interrupteur entraîne le fonctionnement du détartreur dentaire à une puissance de détartrage ultrasonique augmentée.

6. Système selon la revendication 1, dans lequel le système est utilisé pour contrôler le fonctionnement d'un appareil de traitement médical.

7. Système selon la revendication 1, dans lequel l'interrupteur au pied transmet un signal « EN FONCTIONNEMENT » et un signal « ÉTAT DE POSITION D'INTERRUPTEUR » à l'appareil lors de l'activation du premier interrupteur dans l'interrupteur au pied, et l'appareil répond aux signaux en fonctionnant dans un premier mode.

8. Système selon la revendication 1, dans lequel l'interrupteur au pied transmet un signal « EN FONCTIONNEMENT » et un signal « ÉTAT DE POSITION D'INTERRUPTEUR » à l'appareil lors de l'activation du second interrupteur dans l'interrupteur au pied, et l'appareil répond aux signaux en fonctionnant dans un second mode.

9. Système selon la revendication 1, dans lequel l'appareil transmet un signal « DEMANDE D'ÉTAT DE POSITION D'INTERRUPTEUR » à l'interrupteur au pied à des intervalles de temps de 250 millisecondes.

10. Système selon la revendication 1, dans lequel l'interrupteur au pied est alimenté par batterie.

11. Système selon la revendication 10, dans lequel l'interrupteur au pied transmet un signal « EN FONCTIONNEMENT » et l'unité de base répond en transmettant un signal « RECONNAISSANCE » et un signal « DEMANDE D'ÉTAT DE LA BATTERIE » à l'interrupteur au pied.

12. Système selon la revendication 11, dans lequel l'interrupteur à pied répond au signal « DEMANDE D'ÉTAT DE LA BATTERIE » en transmettant un signal « RECONNAISSANCE » et des données de tension de la batterie à l'appareil.

13. Système selon la revendication 1, dans lequel l'interrupteur au pied transmet des signaux opérationnels de manière sans fil à l'appareil de traitement dentaire ou médical, jusqu'à ce que l'appareil transmette un signal DEMANDE DE MODE VEILLE à l'interrupteur au pied et que l'interrupteur au pied réponde en entrant dans un mode veille, les signaux de fonctionnement sans fil étant ignorés par l'appareil.

14. Système selon la revendication 13, dans lequel le système est utilisé pour contrôler le fonctionnement d'un appareil de traitement dentaire.

15. Système selon la revendication 14, dans lequel l'appareil de traitement dentaire est un détartreur dentaire à ultrasons.
